# EUROPÄISCHE PATENTANMELDUNG

(11) **EP 0 878 534 A2**
(43) Veröffentlichungstag der Anmeldung: **18.11.1998**
(21) Anmeldenummer: 98104688.1
(22) Anmeldetag: 16.03.1998
(51) Int. Cl.: C11B 3/00, C11C 3/12, C07C 51/36

(54) **Verfahren zur kontinuierlichen katalytischen Umsetzung von organischen Verbindungen**

(30) Priorität: 12.05.1997 DE 19719431
(71) Anmelder: DEGUSSA AG, 60311 Frankfurt (DE)
(72) Erfinder: Tacke, Thomas, Dr., 61381 Friedrichsdorf (DE); Röder, Stefan, 36391 Sinntal (DE); Beul, Inge, 63584 Gründau 2 (DE); Laporte, Steffen, 63110 Rodgau (DE); Panster, Peter, Dr., 63517 Rodenbach (DE)

(57) **Zusammenfassung**

Die Erfindung betrifft eine Verfahren zur kontinuierlichen katalytischen Umsetzung einer oder mehrerer organischer Verbindungen, die zusammen mit unerwünschten Begleitstoffen einen Ausgangsstoff bilden: Zunächst werden die gewünschten organischen Verbindungen des Ausgangsstoffes mittels verdichteter Fluide gezielt extrahiert. Dann wird das Extrakt aus den verdichteten Fluiden und Organischen Verbindungen als Reaktionsgemisch, gegebenenfalls unter Zugabe weiterer Reaktanden, über einen Katalysator geleitet, an dem die katalytische Umsetzung der organischen Verbindungen zu einem Produktgemisch stattfindet, welches aus den Einzelprodukten der katalytischen Umsetzung besteht. Das Produktgemisch wird vom Reaktionsgemisch abgetrennt und die verwendeten Fluide gegebenenfalls zur Extraktion zurückgeführt.

## Beschreibung

Die Erfindung betrifft ein Verfahren zur katalytischen Umsetzung einer oder mehrerer organischer Verbindungen, die zusammen mit unerwünschten Begleitstoffen einen Ausgangsstoff bilden.

In der Chemie besteht häufig die Notwendigkeit, eine katalytische Umsetzung an einem Gemisch von organischen Verbindungen durchzuführen, das neben den gewünschten Ausgangsstoffen Begleitstoffe enthält, die entweder die katalytische Umsetzung stören oder im Endprodukt unerwünscht sind. Bei den katalytischen Umsetzungen, die hier in Betracht kommen handelt es sich zum Beispiel um Alkylierungen, Acylierungen, Veresterungen, Umesterungen, Oxidationen oder Hydrierungen. Die Ausgangsstoffe können natürlichen oder synthetischen Ursprungs sein.

Ein technisch sehr wichtiges Beispiel einer solchen Umsetzung ist die katalytische Hydrierung von Fetten, Ölen, Fettsäureestern und freien Fettsäuren aus natürlichen Quellen, die im folgenden auch als Fettrohstoffe bezeichnet werden. Ziel der Hydrierung dieser organischen Verbindungen ist es, die in ihnen enthaltenen Doppelbindungen teilweise oder vollständig zu hydrieren, ohne dabei andere funktionelle Gruppen, wie zum Beispiel die Carboxylgruppe, der Verbindungen anzugreifen. Die vollständige Hydrierung dieser Verbindungen wird auch als Härtung bezeichnet, da sich hierdurch ihr Schmelzpunkt erhöht. Soll gezielt nur eine bestimmte Anzahl der Doppelbindungen hydriert werden, so spricht man von selektiver Hydrierung. Die Hydrierung erfolgt katalytisch mit Hilfe von Wasserstoff.

Fette und Öle aus natürlichen Quellen enthalten Begleitstoffe, die sowohl bei ihrer späteren Verwendung unerwünscht sind als auch als Katalysatorgifte wirken und zu einer schnellen Desaktivierung der Hydrierkatalysatoren führen. Als Katalysatorgifte werden im Rahmen dieser Erfindung alle Stoffe bezeichnet - unabhängig von ihrer chemischen Natur oder ihres Ursprungs - die die Katalysatoraktivität herabsetzen. Es handelt sich dabei um natürliche Inhaltsstoffe von Fetten und Ölen, um Zersetzungsprodukte oder aber um Stoffe, die während der Verarbeitung eingeführt werden (H. Klimmek, JAOCS, Vol. 61, No. 2, Febr. 1984). Insbesondere gehören hierzu Schwefel-, Phosphor-, Chlor- und Stickstoffverbindungen sowie zum Beispiel oxidierte Fettsäuren, Seifen und Wasser.

Vor der Hydrierung der Fette, Öle, Fettsäureester und freien Fettsäuren werden die Ausgangsstoffe daher in einem separaten Schritt von den unerwünschten Begleitstoffen befreit. Dies geschieht bei Fetten und Ölen durch eine chemische beziehungsweise physikalische Raffination, und bei freien Fettsäuren gewöhnlich durch Vakuumdestillation. Je nach gewünschtem Reinheitsgrad für die Ausgangsstoffe kann das Reinigungsverfahren mehrstufig ausgeführt werden. Bei der Vakuumdestillation zur Reinigung von freien Fettsäuren können allerdings Abbauprodukte entstehen, da die eingesetzten Fettrohstoffe thermisch leicht zersetzlich sind. Die Abbauprodukte verursachen häufig einen unangenehmen Geruch der destillierten Produkte, der durch Desodorierung beseitigt werden muß. Die Desodorierung wird erst nach der Hydrierung vorgenommen. Aufgrund der thermischen Empfindlichkeit der Produkte ist neben einer kurzen Verweilzeit insbesondere eine niedrige Temperatur bei der Verarbeitung möglichst einzuhalten.

Seit etwa 1970 werden verdichtete Gase zur Extraktion, Raffination, Desodorierung und Fraktionierung von Fettrohstoffen eingesetzt, so zum Beispiel in DE 23 63 418 C3, US 4,156,688, DE 35 42 932 A1, DE 42 33 911, DE 43 26 399 C2, EP 0 721 980 A2 und DE 44 47 116 A1. Diese Verfahren beschreiben den Einsatz verdichteter Gase im unterkritischen (verflüssigten), nahekritischen und überkritischen Zustand, wobei im Vergleich zur Destillation unter relativ schonenden Verfahrensbedingungen gearbeitet wird.

Speiseöle und freie Fettsäuren werden heute noch zu über 99% chargenweise in Rühr- oder Schleifenreaktoren gehärtet. Hierbei werden pulverförmige Nickel-Kieselgur-Katalysatoren zur Hydrierung eingesetzt, die nach der Hydrierung durch Filtration aus dem Produkt entfernt werden müssen. Nachteilig bei diesem Verfahren sind niedrige Raum-Zeit-Ausbeuten und die Bildung unerwünschter Nebenprodukte als Folge des durch Diffusion aus der Gasphase über die Flüssigphase zum Katalysator begrenzten Transportes von Wasserstoff. Darüber hinaus weisen diese Verfahren hohe Kosten zum Beispiel für Personal, Energie und Filtration auf. Die Filtration setzt die Produktausbeute herab, da gehärtetes Produkt im Filterrückstand verbleibt.

Der Nickel-Kieselgur-Katalysator bildet bei der Härtung von Speiseöl einen erheblichen Anteil sogenannter trans-Fettsäuren. Diese Tatsache ist von besonderem Nachteil, da trans-Fettsäuren im Verdacht stehen, den Fettspiegel und Cholesteringehalt im menschlichen Blut zu erhöhen.

Bei der Durchhärtung von freien Fettsäuren für oleochemische Anwendungen desaktiviert der Nickel-Kieselgur-Katalysator durch die Bildung von sogenannten Nickelseifen. Diese verbleiben im Produkt und müssen durch Destillation abgetrennt werden. Nickelseifen stellen ein Abfallprodukt dar und müssen unter erheblichen Kosten deponiert werden.

Zur Vermeidung der zuvor genannten Nachteile der chargenweisen Härtung beziehungsweise des Einsatzes von Nickel-Kieselgur-Katalysatoren wurden kontinuierliche Verfahren entwickelt, bei denen Palladium-Festbettkatalysatoren zum Einsatz kommen. Den Stand der Technik hierzu beschreiben die Patente beziehungswiese Patentanmeldungen CA 1 157 844, DE 41 09 502 C2 und DE 42 09 832 A1 bzw. EP 0 632 747 B1.

Gemäß der DE 41 09 502 C2 wird die kontinuierliche Härtung von Rohfettsäuren im Rieselbett an einem Palladium/Titanoxid-Katalysator vorgenommen. Die Reaktionsmedien werden dabei in Form eines 2-Phasen-Gemisches aus flüssigen Fettsäuren und Wasserstoffgas am Festbettkatalysator zur Reaktion gebracht. Die Hydrieraktivität in diesem Verfahren läßt dabei nur Raumgeschwindigkeiten von 1,2 h⁻¹ zu. Der in diesem Verfahren verwendete Katalysator weist eine bedingte Resistenz gegenüber den in den Fettrohstoffen enthaltenen Katalysatorgiften auf. Auf eine separate Reinigung der Ausgangsstoffe kann aber auch hier bei großtechnischer Anwendung des Katalysators zur Verminderung des Katalysatorverbrauchs nicht verzichtet werden.

Die WO 95/22591 und WO 96/01304 beschreiben Verfahren, in denen überkritische Fluide als Lösungsmittel für Fette, Öle, freie Fettsäuren und Fettsäureester und Wasserstoff eingesetzt werden. Gemäß der WO 95/22591 werden dabei die genannten Verbindungen mit dem für die Hydrierung benötigten Wasserstoff und in Gegenwart eines überkritischen Fluids an einem Katalysator umgesetzt und anschließend durch Entspannen des überkritischen Fluids von diesem abgetrennt. Die überkritischen Fluide ermöglichen in diesem Verfahren einen verbesserten Stofftransport insbesondere für Wasserstoff und einen verbesserten Wärmeaustausch. Darüber hinaus erniedrigen sie die Viskosität des Reaktionsmediums, so daß deutlich erhöhte Raum-Zeit-Ausbeuten und verbesserte Selektivitäten erzielt werden können. Über die notwendige Reinheit der Ausgangsstoffe für dieses Verfahren werden keine Aussagen gemacht.

Durch die der Hydrierung in der Regel vorgeschalteten Reinigungsschritte gelingt es, sowohl in der chargenweisen Härtung mit Nickel-Kieselgur-Katalysator als auch in der kontinuierlichen Härtung in Gegenwart überkritischer Fluide, den Katalysatorverbrauch pro gehärteter Tonne Fettsäure deutlich zu senken.

Die US 3,969,382 beschreibt die simultane Hydrierung und Desodorierung von Fetten und Ölen in Gegenwart von überkritischem Kohlendioxid, Wasserstoff und einem feinteiligen Nickel-Hydrierkatalysator bei Temperaturen von 100 bis 250°C und einem Druck von 150 bis 300 bar. Der Katalysator wird nach der Hydrierung über eine Filterpresse von den gehärteten Produkten abgetrennt. Die simultane Hydrierung und Desodorierung hat den Nachteil, daß der Katalysator mit den Katalysatorgiften direkt in Kontakt kommt und schnell desaktiviert.

Der Stand der Technik zur Hydrierung von Fettrohstoffen beschreibt also chargenweise oder kontinuierliche Verfahren, die jedoch nur bedingt gegenüber den in den Ausgangsstoffen enthaltenen Katalysatorgiften unempfindlich sind und in der Regel die Reinigung der Ausgangsstoffe in einem separaten Reinigungsschritt erfordern. Ähnliche Verhältnisse liegen bei den anderen eingangs angeführten Verfahren vor.

Aufgabe der vorliegenden Erfindung ist es daher, ein Verfahren zur katalytischen Umsetzung von organischen Verbindungen anzugeben, welches die Reinigung des Ausgangsstoffes und die katalytische Umsetzung der organischen Verbindungen in einem einzigen Verfahren ermöglicht.

Zur Lösung dieser Aufgabe wird das folgende kontinuierliche Verfahren zur katalytischen Umsetzung einer oder mehrerer organischer Verbindungen vorgeschlagen, die zusammen mit unerwünschten Begleitstoffen einen Ausgangsstoff bilden: Zunächst werden die gewünschten organischen Verbindungen des Ausgangsstoffes mittels verdichteter Fluide gezielt extrahiert. Das Extrakt aus den verdichteten Fluiden und organischen Verbindungen wird als Reaktionsgemisch, gegebenenfalls unter Zugabe weiterer Reaktanden, über einen Katalysator geleitet, an dem die katalytische Umsetzung der organischen Verbindungen zu einem Produktgemisch stattfindet, welches aus den Einzelprodukten der katalytischen Umsetzung besteht. Das Produktgemisch wird vom Reaktionsgemisch abgetrennt und die verwendeten Fluide gegebenenfalls zur Extraktion zurückgeführt.

Unter dem Begriff 'verdichtete Fluide' werden im Rahmen dieser Erfindung, mit Ausnahme von Wasserstoff, Fluide verstanden, die unter den gewählten Extraktions- und Reaktionsbedingungen (Temperatur und Druck) entweder einen Dampfdruck aufweisen, der geringer als der gewählte Druck ist und die somit im verflüssigten Zustand vorliegen, oder die unter den gewählten Reaktionsbedingungen sich im nahekritischen, kritischen oder überkritischen Zustand befinden. Mit nahekritisch sind hier Zustandsbedingungen gemeint, bei denen die gewählte Temperatur T kleiner als die kritische Temperatur Tₖ (T<Tₖ) und der gewählte Druck p größer oder gleich dem kritischen Druck pₖ (p≥pₖ) sind.

Bevorzugt werden Extraktion und katalytische Umsetzung bei Temperaturen zwischen 0 und 300°C und Drücken zwischen 10 und 800 bar vorgenommen.

Die verdichteten Fluide dienen im erfindungsgemäßen Verfahren als Lösungsmittel für die organischen Verbindungen und für die weiteren Reaktanden. Es werden daher solche Fluide gewählt, die unter den Extraktions- und Reaktionsbedingungen des Verfahrens ein gutes Lösevermögen für diese Stoffe aufweisen. Als besonders geeignete Fluide haben sich hierbei Butan, Ethan, Kohlendioxid, Propan, Distickstoffmonoxid oder Mischungen davon erwiesen.

Die Extraktion wird bevorzugt im Gegenstrom mit den genannten Fluiden vorgenommen. Sie kann in üblichen Extraktoren durchgeführt werden. Hierbei werden die organischen Verbindungen in den verdichteten Fluiden gelöst und als Extrakt über Kopf des Extraktors abgezogen, während die unerwünschten Begleitstoffe, bei denen es sich um organische Verbindungen insbesondere mit Heteroatomen (P, S, N) und um anorganische Verbindungen handelt, schlechter löslich sind und sich im Extraktionssumpf sammeln. Hier werden sie von Zeit zu Zeit abgezogen.

Zur Erhöhung der Reinheit der organischen Verbindungen kann die Extraktion mehrstufig durchgeführt werden. Eine weitere Verbesserung der Extraktion wird durch Zugabe sogenannter Modifikatoren zu den verdichteten Fluiden erhalten. Mit Modifikatoren werden Stoffe bezeichnet, die die Löslichkeit der organischen Verbindungen in den verdichteten Fluiden erhöhen. Geeignete Modifikatoren sind polare organische Verbindungen wie z. B. Aceton und/oder C₁ - C₆-Alkohole.

Das Extrakt besteht aus den verdichteten Fluiden und den darin gelösten organischen Verbindungen. Je nach durchzuführender katalytischer Reaktion wird das Extrakt direkt als Reaktionsgemisch über einen geeigneten Katalysator geleitet oder erst nach Zugabe eines weiteren Reaktanden wie zum Beispiel Sauerstoff für Oxidationsreaktionen oder Wasserstoff für Hydrierungen. Gegebenenfalls können hier auch weitere verdichtete Fluide zugemischt werden. Am Katalysator werden die im Reaktionsgemisch enthaltenen organischen Verbindungen umgesetzt. Je nach dem Spektrum der organischen Verbindungen im Ausgangsstoff (zum Beispiel freie Fettsäuren mit verschiedenen Kettenlängen) wird durch die katalytische Umsetzung ein Produktgemisch aus verschiedenen Einzelprodukten erhalten.

Bei der Durchführung von Hydrierreaktionen wird Wasserstoff gegebenenfalls in einem mehrfachen stöchiometrischen Überschuß angewendet, um die katalytisch aktiven Zentren des Katalysators ausreichend mit Wasserstoff zu versorgen. Da das Lösungsvermögen der Fluide in der Regel zur Aufnahme der gesamten Wasserstoffmenge nicht ausreicht, werden entweder parallel zum Wasserstoff weitere überkritische Fluide hinzugefügt, um eine vollständige Löslichkeit des Wasserstoffs im Fluid zu ermöglichen, oder der überschüssige Wasserstoff wird als verdichtetes Gas zusammen mit dem Reaktionsgemisch über den Katalysator geleitet.

Das Abtrennen des Produktgemisches vom Reaktionsgemisch kann auf einfache Weise durch Vermindern des Druckes oder durch Erhöhen der Temperatur vorgenommen werden. Der Druck wird dabei zum Beispiel so weit herabgesetzt, daß die Fluide in die Gasphase übergehen und das Produktgemisch zurückbleibt. Alternativ hierzu kann durch Erhöhen der Temperatur die Löslichkeit des Produktgemisches in dem Reaktionsgemisch herabgesetzt werden, so daß ebenfalls eine Abtrennung des Produktgemisches vom Reaktionsgemisch erfolgt. Bei der Abtrennung des Produktgemisches kann dieses auch gleich in seine Einzelprodukte durch entsprechend fraktionierte Druckverminderung beziehungsweise Temperaturerhöhung aufgetrennt werden.

Ein bevorzugtes Einsatzgebiet des erfindungsgemäßen Verfahrens ist die selektive oder vollständige Hydrierung von Fetten, Ölen, Fettsäureestern und freien Fettsäuren. Im Unterschied zur US 3,969,382 wird im erfindungsgemäßen Verfahren der Ort der Reinigung des Ausgangsstoffes durch Extraktion vom Ort der Hydrierung getrennt. Im erfindungsgemäßen Verfahren kommen ein in Reihe geschalteter Extraktor und ein Hydrierreaktor zum Einsatz. Zunächst wird der Ausgangsstoff gereinigt. Er wird dann im gereinigten Zustand im Hydrierreaktor umgesetzt. Dadurch lassen sich deutlich verbesserte Standzeiten des Katalysators erzielen. Werden dagegen Hydrierung und Desodorierung von Fetten und Ölen wie in der US 3,969,382 simultan durchgeführt, so kommt der Katalysator in Kontakt mit Katalysatorgiften. Die Folge sind verringerte Standzeiten des verwendeten Katalysators.

Für die Hydrierung von Fetten, Ölen, Fettsäureestern und freien Fettsäuren können alle bekannten Hydrierkatalysatoren eingesetzt werden, wie zum Beispiel Nickel-, Platin-, Palladium-, Rhodium-, Ruthenium-Katalysatoren oder Kombinationen hiervon auf Kieselsäure bzw. Siliziumdioxid, Aluminiumoxid, Titanoxid, Zirkonoxid, Magnesiumoxid, Aktivkohle oder Mischoxiden wie Magnesiumaluminat. Besonders bewährt haben sich die Platingruppenmetalle auf geformten Trägern. Die katalytische Aktivität kann durch Promotoren beeinflußt werden. So ist zum Beispiel bekannt, daß Silber als Promotor für Nickel- und Palladium-Katalysatoren die Bildung von trans-Isomeren vermindert.

Die Träger sollten eine hohe spezifische Oberfläche aufweisen, um eine gute Dispersion der Katalysatormetalle zu ermöglichen. Vorteilhaft sind spezifische Oberflächen zwischen 10 und 1000 m²/g. Besonders wichtig ist auch die Porenstruktur der Träger. Sie sollten ein Gesamtporenvolumen zwischen 0,05 und 6,5 ml/g aufweisen, welches sich überwiegend aus Meso- und Makroporen zusammensetzt. Mikroporen sind unerwünscht und sollten nur einen geringen Prozentsatz am Gesamtporenvolumen ausmachen.

Die Begriffe Mikro-, Meso- und Makroporen werden hier in Übereinstimmung mit den Definitionen der IUPAC verwendet. Gemäß diesen Definitionen umfassen die Porengruppen folgende Durchmesserbereiche:
- Mikroporen:: d < 2 nm
- Mesoporen:: d = 2 ... 50 nm
- Makroporen:: d > 50 nm

Meso- und Makroporen garantieren durch ihre großen Porendurchmesser eine optimale Zugänglichkeit der auf ihren Oberflächen abgeschiedenen katalytisch aktiven Edelmetallkristalle für die Fett-, Fettsäure- bzw. FettsäureesterMoleküle. Unterstützt wird diese Zugänglichkeit durch die Tatsache, daß die verwendeten verdichteten Fluide eine geringe Viskosität aufweisen.

Der Gehalt an Platingruppenmetallen auf dem Träger sollte zwischen 0,05 und 5 Gew.-% betragen, bevorzugt zwischen 0,1 und 3,0 Gew.-%.

Die Platingruppenmetalle müssen auf dem Träger fein verteilt abgeschieden werden, um eine möglichst große Metalloberfläche für den katalytischen Prozeß zur Verfügung zu stellen. Ein Maß für die Größe der katalytisch aktiven Metalloberfläche ist die Adsorption von Kohlenmonoxid. Sie sollte in Abhängigkeit vom Gehalt an Platingruppenmetallen zwischen 0,05 und 5,0 ml CO/g der fertigen Katalysatorkörper liegen.

Die Katalysatorträger können beliebig geformt sein. Geeignet sind insbesondere alle für Festbettkatalysatoren bekannten Formen, also Kugeln, Zylinder, Hohlzylinder und Speichenräder sowie monolithische Katalysatorträger in Form von Wabenkörpern mit parallelen Strömungskanälen oder Schaumkeramiken mit einem offenen Porensystem. Die monolithischen Wabenkörper können durchgängig aus dem hochoberflächigen Trägermaterial bestehen (Vollkatalysator) oder aus einem inerten Tragkörper mit einer Beschichtung aus dem hochoberflächigen Trägermaterial aufgebaut sein (Beschichtungskatalysator).

Durch die geringe Viskosität der verdichteten Fluide ist es möglich, relativ kleinteilige Katalysatorträger als Schüttgut einzusetzen, ohne daß der Druckabfall über das Katalysatorbett zu groß wird. Vorteilhaft sind Katalysatorträger mit äußeren Abmessungen im Bereich zwischen 0,1 und 5,0 mm, insbesondere zwischen 0,2 und 3,0 mm. Dadurch lassen sich sehr hohe Aktivitäten erzielen. Bevorzugt werden kugelförmige Träger verwendet.

Wegen der geringen Abmessungen der Katalysatoren weisen sie in der Schüttung eine sehr hohe geometrische Oberfläche relativ zum Gesamtvolumen der Schüttung auf. Dies kommt der katalytischen Aktivität der Katalysatorschüttung zugute. Weiter verbessert werden kann diese Aktivität, wenn die Platingruppenmetalle auf diesen Trägern in einer äußeren Schale von 10 - 40 µm aufgebracht sind. Die Schalenimprägnierung ist besonders für die selektive Fetthärtung von Bedeutung. Sie verhindert nämlich, daß Fettmoleküle, die in das Innere des Katalysatorträgers hineindiffundiert sind, dort lange mit katalytisch aktiven Metallen in Berührung stehen und somit voll durchgehärtet werden. Für die vollständige Härtung von Fetten bzw. Fettsäuren können dagegen auch voll durchimprägnierte Katalysatorträger eingesetzt werden.

Als Katalysatorträger eignen sich verschiedene Materialien. Sie müssen allerdings die obengenannten Forderungen an ihre physikalischen Eigenschaften erfüllen und gegenüber den Reaktionsmedien, insbesondere gegenüber den Fettsäuren, beständig sein. Bei der konventionellen Fetthärtung haben sich Aktivkohle, Siliziumdioxid, Aluminiumoxid, Aluminium/Silizium-Mischoxide, Bariumsulfat, Titanoxid, mit Titanoxid beschichtete Glasperlen und Ionenaustauscherharze bewährt. Diese Trägermaterialien können auch im erfindungsgemäßen Verfahren eingesetzt werden. In optimaler Weise werden die genannten Forderungen aber von Organosiloxanamin-Copolykondensaten oder von polymeren, sekundären und/oder tertiären Organosiloxanamin-Verbindungen oder von Organosiloxan-Polykondensaten erfüllt. Diese Trägermaterialien werden in den deutschen Patentschriften DE 38 00 563 C1, DE 38 00 564 C1, DE 39 25 359 C1, DE 39 25 360 C1 und DE 42 25 978 C1 beschrieben. Die Patentschriften DE 41 10 705 C1 und DE 41 10 706 C1 offenbaren Katalysatoren auf Basis von Platingruppenmetallen.

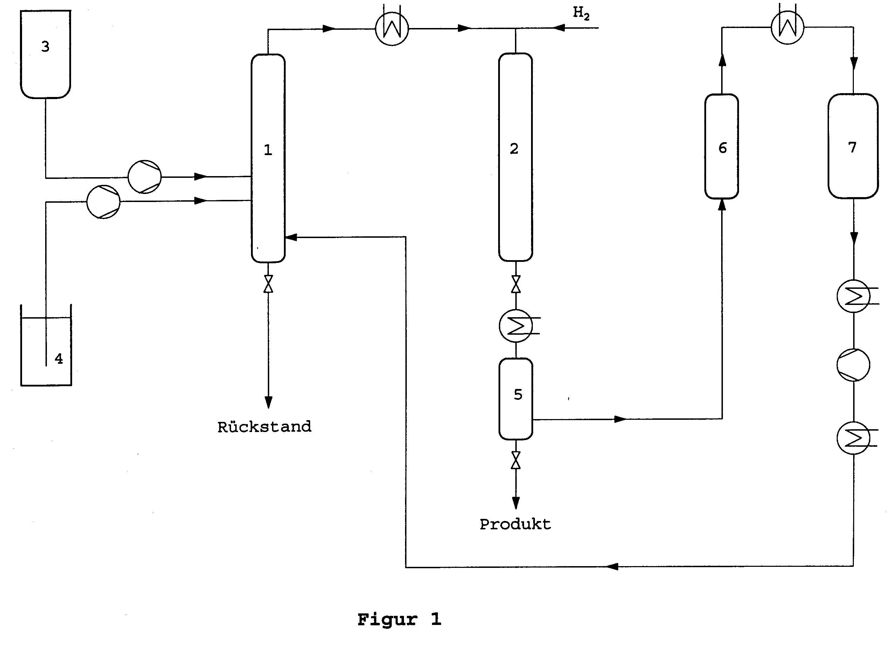

Die folgenden Beispiele dienen der weiteren Erläuterung der Erfindung. Figur 1 zeigt die schematische Darstellung der für die Beispiele benutzten Testapparatur. Bezugsziffer 1 bezeichnet die Extraktionskolonne. Es handelt sich um ein 1000 mm langes Edelstahlrohr mit einem Innendurchmesser von 30 mm, gefüllt mit sogenannten 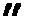 Sulzer- Packungen" CY aus Metallgewebe. Die Kolonne wird im Gegenstromverfahren betrieben. Die verdichteten Fluide dienen als Extraktionsmittel und werden der Kolonne von unten zugeführt und auf einer Höhe von 120 mm über einen Teller verteilt. Im unteren verbleibenden Raum kann sich der entstehende Rückstand sammeln (Extraktionssumpf) und diskontinuierlich abgelassen werden. Der Extraktstrom wird am Kopf des Extraktors entnommen. Der Ausgangsstoff befindet sich im Vorratsbehälter 3 und wird der Extraktionskolonne in einer Höhe von 420 mm zugeführt, so daß sich die Extraktionsapparatur in einen 300 mm langen 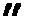 Abtriebsteil" (reine Extraktion) und einen 580 mm langen 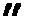 Verstärkerteil" (Anreicherung des Extraktes) unterteilt. Zur Beeinflussung des Lösevermögens des Extraktionsmittels kann ein im Behälter 4 befindlicher Modifikator eingesetzt werden. Der Modifikator wird entweder separat oder zusammen mit dem Ausgangsstoff oder dem Extraktionsmittel der Extraktionskolonne zugeführt.

Der Extraktor wird mit mehreren Heizungen elektrisch beheizt, so daß verschiedene Temperaturgradienten längs des Extraktors einstellbar sind. Insbesondere kann der Extraktorkopf, das heißt ein Bereich von etwa 100 mm Länge, stärker beheizt werden, als die restliche Extraktionskolonne. Hierdurch wird die Löslichkeit der extrahierten Stoffe im Extraktionsmittel vermindert und ein interner Extraktrücklauf in der Kolonne eingestellt. Ähnlich einer Rektifikation kann so die Reinheit des Extraktes zusätzlich gesteigert werden.

Das Extrakt gelangt von oben in den Hydrierreaktor 2, der von einem 750 mm langen Edelstahlrohr mit einem Innendurchmesser von 15 mm gebildet wird. Dieses Edelstahlrohr ist im unteren Drittel mit einem Katalysatorvolumen von 5 ml befüllt. Ober- und unterhalb der Katalysatorschüttung befinden sich Stopfen aus Quarzwolle. Sie trennen das eigentliche Katalysatorbett von Glasperlen, die das freie verbleibende Volumen des Edelstahlrohres ober- und unterhalb der Katalysatorschüttung auffüllen. Die Inertschüttung oberhalb der Katalysatorschüttung dient gleichzeitig als statischer Mischer zum Vermischen der verschiedenen Medien und Reaktanden. Der Reaktor wird elektrisch beheizt. Der für die Hydrierung benötigte Wasserstoff wird zusammen mit dem Extrakt dem Reaktor von oben zuführt. Extrakt und Wasserstoff bilden das Reaktionsgemisch.

Nach Durchlaufen des Reaktors gelangt das Reaktionsgemisch in einen Separator 5. Im Separator wird das Reaktionsgemisch durch Druckerniedrigung unterhalb des kritischen Druckes in das flüssige Produktgemisch und gasförmiges Fluid und restlichen Wasserstoff aufgetrennt. Das abgetrennte Fluid und der restliche Wasserstoff können entweder an die Atmosphäre abgegeben werden oder wieder verflüssigt beziehungsweise komprimiert zur Extraktion zurückgeführt werden. Zu diesem Zweck wird das Gasgemisch über einen Adsorber 6 zur Entfernung eventuell aus dem Separator ausgetragenen Produktes geleitet und im Pufferbehälter 7 zwischengespeichert.

Alle Leitungen der Testapparatur und die Vorratsbehälter für die eingesetzten Fettsäuren sind elektrisch beheizt.

Mit der Testapparatur von Figur 1 wurden zunächst Vorversuche zur Gegenstromextraktion von Rohfettsäure mit verschiedenen Fluiden unternommen. Das Extrakt und der Extraktionssumpf wurden dabei in festgelegten Zeitintervallen bezüglich Jodzahl, Säurezahl und Schwefelgehalt untersucht.

Die Jodzahl ist ein Maß für die Anzahl der im Produkt noch nicht abgesättigten Doppelbindungen und wird in Gramm Jod, die von 100 g der Probe absorbiert wird, angegeben. Sie wird gemäß der offiziellen Methode Tgl-64 (Wijs-Methode) des A.O.C.S. bestimmt.

Die Säurezahl (SZ) dient zur Bestimmung des Gehaltes an freien organischen Säuren im Öl ( Vorschrift s. Deutsches Arzneibuch 7. Auflage., 1968). Sie gibt an, wieviel mg KOH nötig sind, um die in 1 g Fett oder Öl enthaltenen freien Säuren zu neutralisieren. Die Säurezahl ist ein Maß für die Reinheit der extrahierten Fettsäuren. Je niedriger die Säurezahl ist, um so mehr Fremdstoffe sind in der Fettsäure enthalten.

Der Schwefelgehalt wurde über einen Wickbold-Aufschluß der Fettsäure und anschließender Ionenchromatographie ermittelt. Der Schwefelgehalt dient als Maßzahl für die in der Fettsäure enthaltenen Katalysatorgifte, wie zum Beispiel Schwefel-, Phosphor-, Stickstoff- und Chlor-Verbindungen.

Die eingesetzte Rohfettsaure bestand im wesentlichen aus Ölsäure und wies die folgenden Kennzahlen auf:

| | |
|---|---|
| Jodzahl: | 56 g Jod/100 g Rohfettsäure |
| Säurezahl: | 187,9 mg KOH/g Rohfettsäure |
| Gehalt an Schwefel: | 84 mg/kg |

Es wurden fünf Extraktionsversuche A bis E mit unterschiedlichen Fluidgemischen und Extraktionsbedingungen vorgenommen. Die gewählten Extraktionsbedingungen sind in Tabelle 1 aufgelistet. Tabelle 2 gibt die Extraktionsergebnisse wieder.

Die kritischen Zustandsgrößen des in diesen Versuchen verwendeten Kohlendioxids und Propans sind gegeben durch:

| | | |
|---|---|---|
| CO₂ : | Tₖ = 31°C; | Pₖ = 73 bar |
| Propan: | Tₖ = 96°C; | Pₖ = 42 bar |

Wie in Tabelle 1 aufgeführt, kann die Rohfettsäure mit CO₂, CO₂/Propan, CO₂/Aceton Gemischen bei 200 bar sowie mit unterkritischem Propan und Propan/CO₂-Gemischen bei 35 bar gereinigt werden.

Mit CO₂ und CO₂/Aceton Gemischen wurden sehr hohe Reinheiten im Extrakt erzielt. Das verdeutlichen auch die niedrigen Schwefelgehalte und die deutlich verbesserten Säurezahlen. Allerdings werden vergleichsweise niedrige Ausbeuten erzielt. Mit Propan/CO₂-Gemischen konnte die Ausbeute sowohl bei 200 bar als auch bei 35 bar Betriebsdruck bei guter Reinheit wesentlich verbessert werden. Mit flüssigem Propan bei 35 bar konnte die höchste Ausbeute erzielt werden.

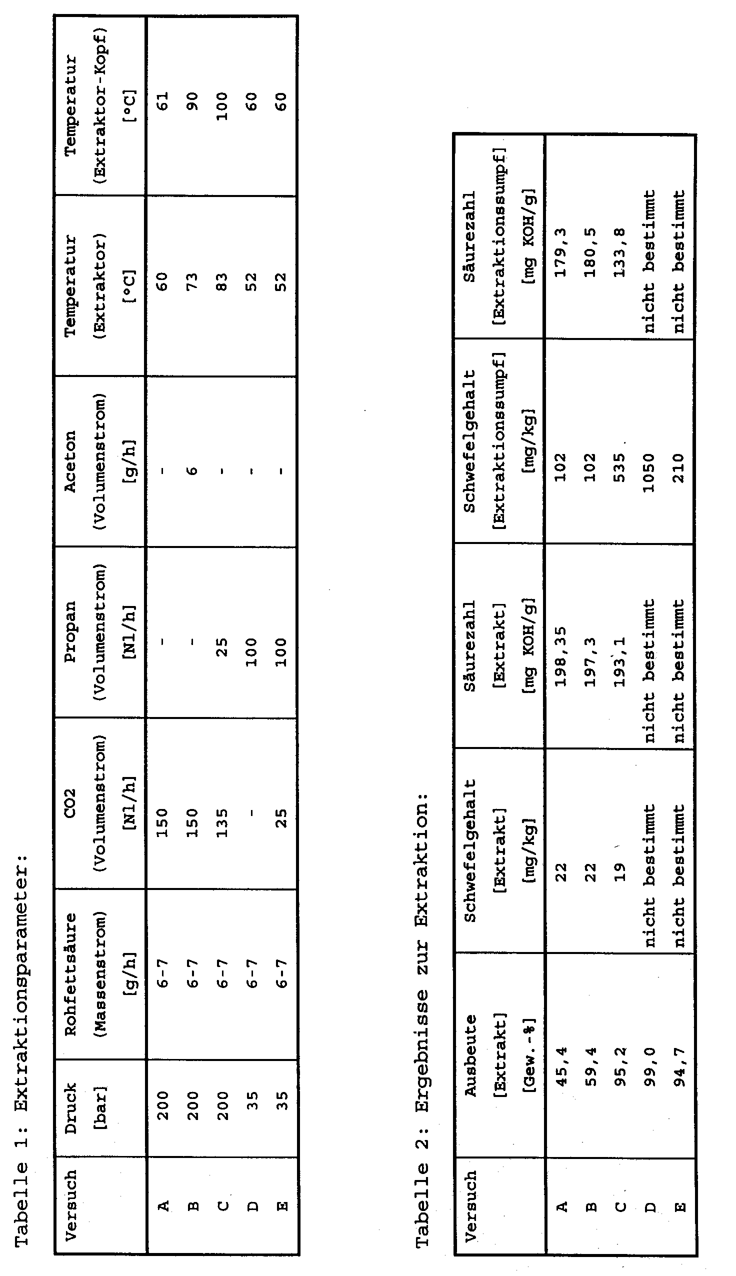

### Beispiel 1:

Die Hydrierung von Rohfettsäure nach dem erfindungsgemäßen Verfahren mit integrierter Reinigung der Rohfettsäure durch Extraktion mit einem CO₂/Propan-Gemisch wurde unter folgenden Verfahrensbedingungen vorgenommen

| | |
|---|---|
| Volumenstrom CO2: | 135 Nl/h |
| Volumenstrom Propan: | 25 Nl/h |
| Volumenstrom H2: | 1 - 100 Nl/h |
| Temperatur im Extraktor | 80°C |
| Temperatur am Extraktorkopf | 100°C |
| Reaktionstemperatur: | 140 - 190 °C |
| Druck: | 200 bar |
| LHSV: | 1 h⁻¹ |

Für die Hydrierung wurde ein Pd/OFP-Katalysator eingesetzt. Pd/OFP bezeichnet einen Palladium-Katalysator auf einem Träger aus einem organofunktionellen Polysiloxan gemäß Beispiel 2 der Patentschrift DE 44 05 029 C2. In dieser Patentschrift sind die Hydriereigenschaften dieses Katalysators in überkritischen Medien sowie seine physikalisch-chemischen Kennzahlen beschrieben.

Die oben angegebene Raumgeschwindigkeit (LHSV = Liquid Hourly Space Velocity) bezieht sich auf das eingesetzte Katalysatorvolumen von 5 ml.

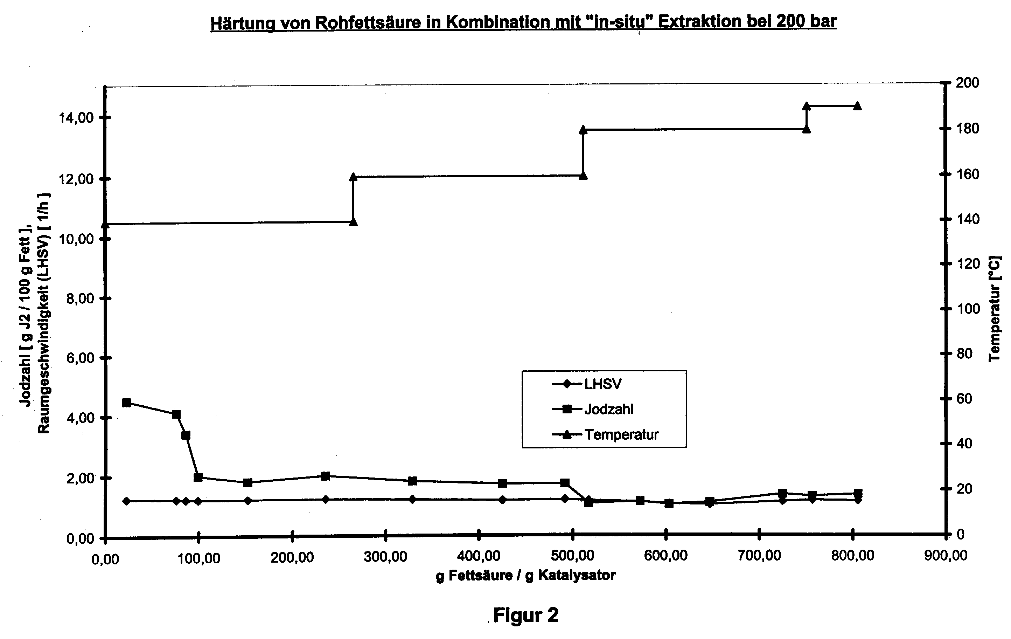

Tabelle 3 und Figur 2 beschreiben den Verfahrensverlauf. Es wurde eine Jodzahl von 1-2 bis zu einem Durchsatz von 800 g Fettsäure pro g Katalysator erzielt. Durch Temperaturerhöhung nach einem Durchsatz von etwa 235 g Fettsäure pro Gramm Katalysator wurde einer möglichen Desaktivierung des Katalysators vorgebeugt und eine weitere Erniedrigung der Jodzahl erreicht.

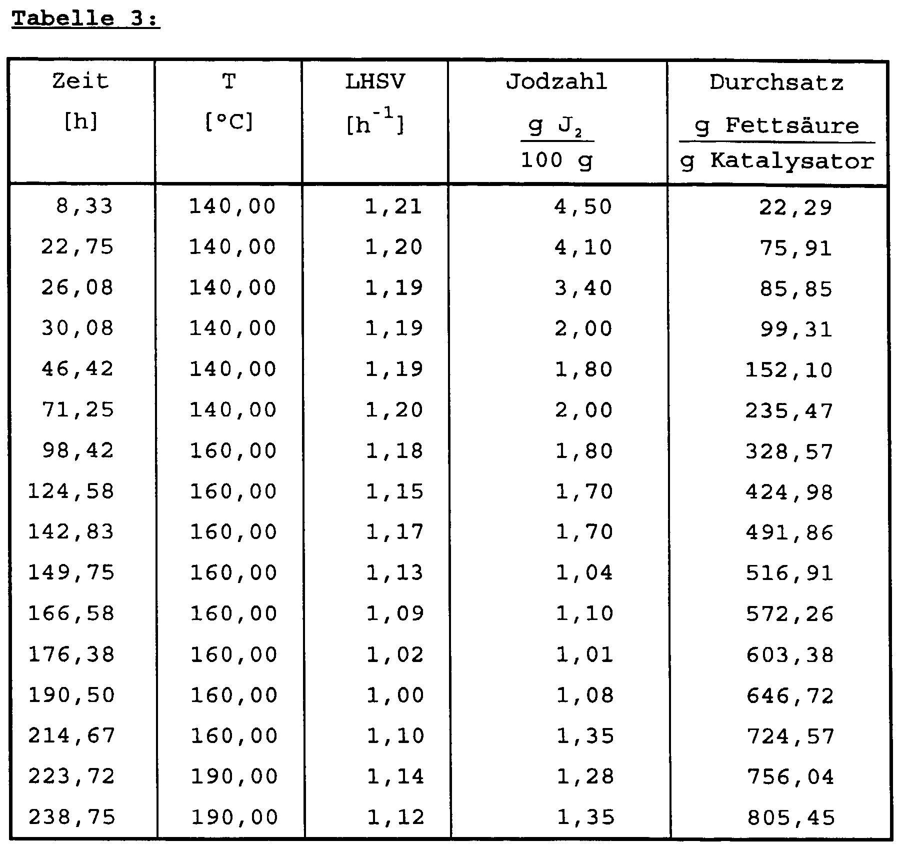

### Vergleichsbeispiel 1:

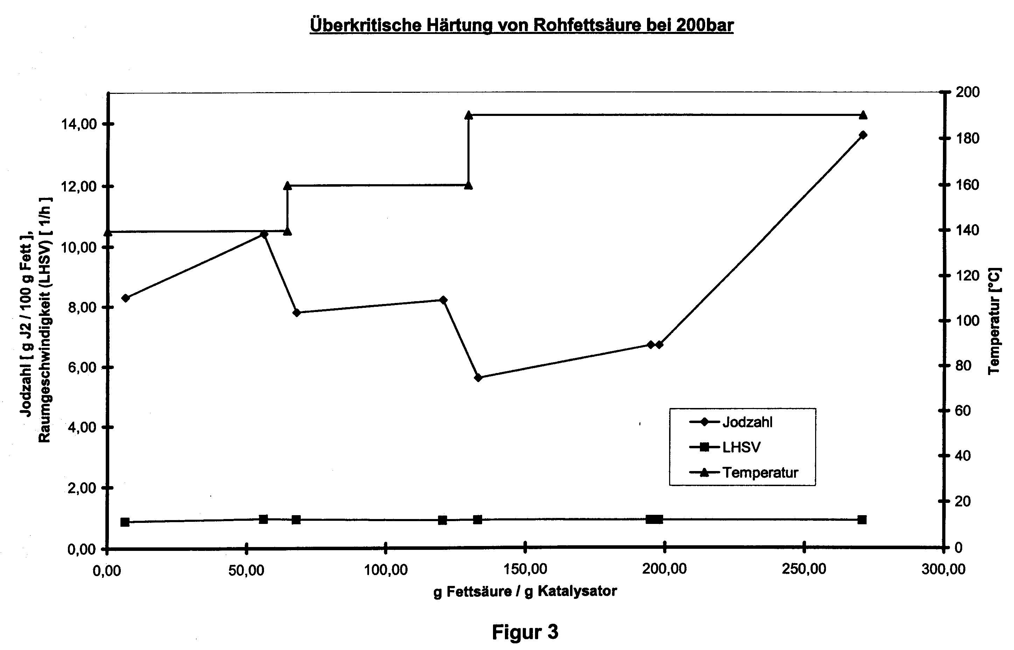

Unter den gleichen Bedingungen wie in Beispiel 1 wurde ein Standzeitversuch mit Rohfettsäure jedoch ohne vorherige Extraktion durchgeführt. Es kamen wiederum 5 ml des 1%-Pd/OFP Katalysators zum Einsatz. Wie in Tabelle 4 und Figur 3 dargestellt, kann selbst bei 190 °C nur eine Jodzahl von 6 erreicht werden. Es tritt eine schnelle Desaktivierung des Katalysators ein.

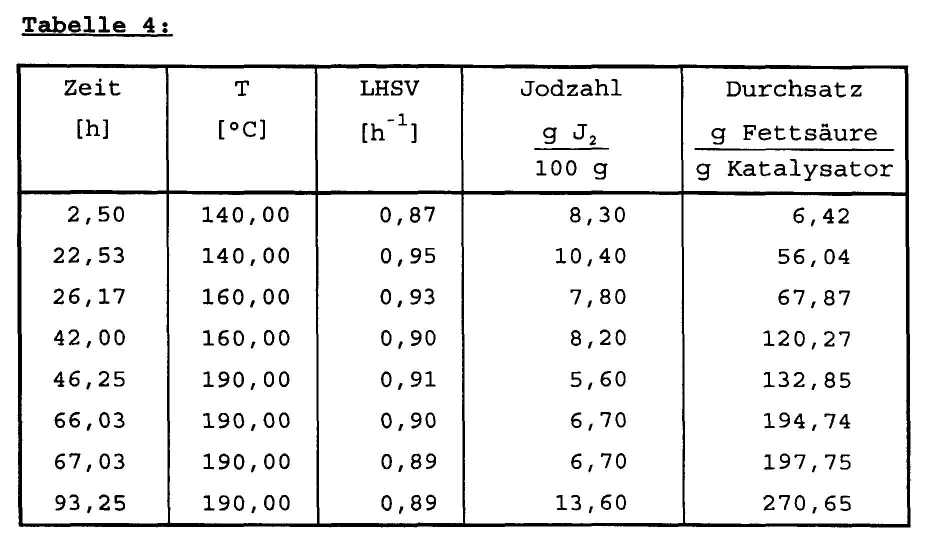

### Vergleichsbeispiel 2:

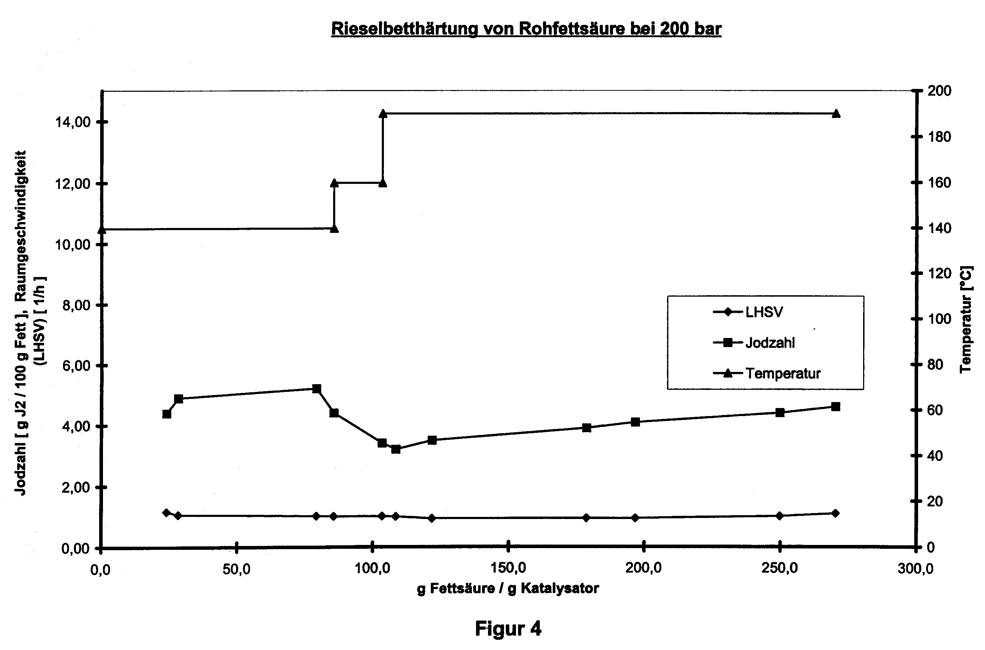

Bei 200 bar wurde eine Vergleichshydrierung mit reinem Wasserstoff in der Rieselbettphase, das heißt ohne Zugabe eines verdichteten Fluids und ohne vorherige Extraktion durchgeführt. Es wurden 5 ml des 1%-Pd/OFP Katalysators bei einem Volumenstrom des Wasserstoffs von 140 Nl/h eingesetzt. Tabelle 5 und Figur 4 beschreiben den Hydrierverlauf. Im Vergleich zu Beispiel 1 werden höhere Jodzahlen und eine schnelle Desaktivierung des Katalysators beobachtet.

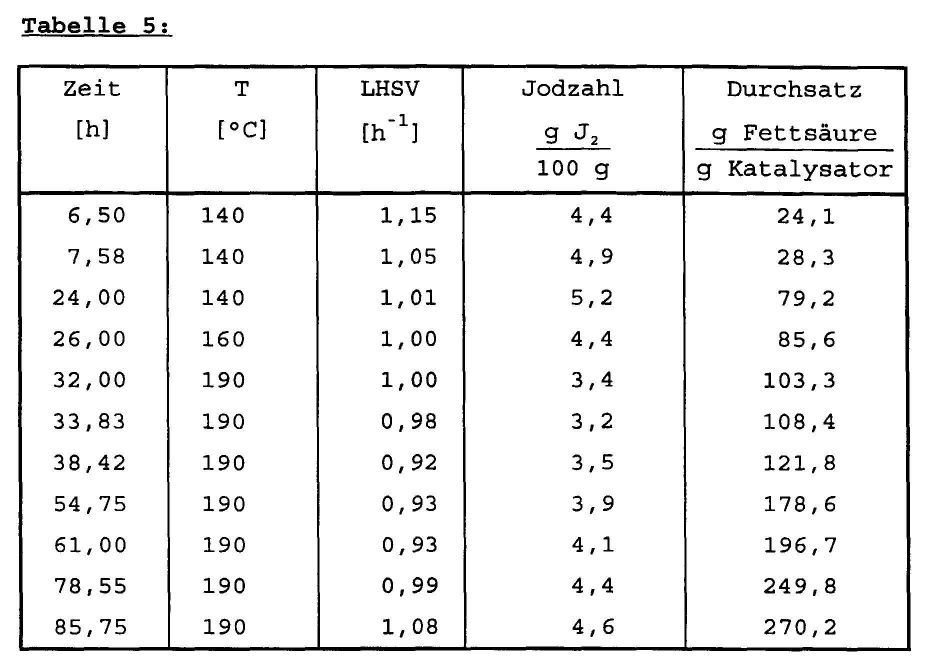

### Vergleichsbeispiel 3:

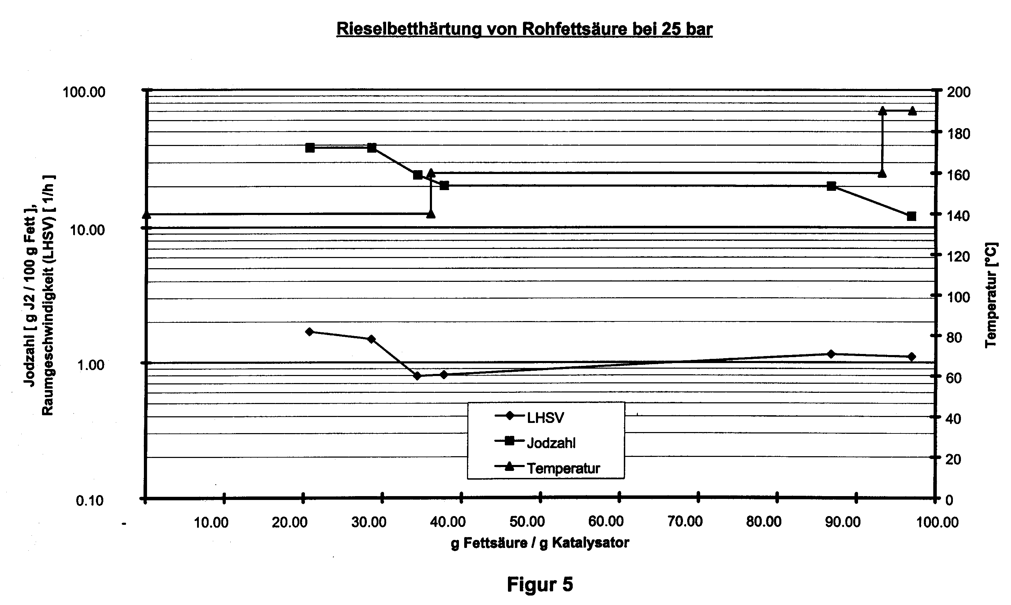

Vergleichsversuch 2 wurde bei einem Wasserstoffdruck von nur 25 bar wiederholt. Tabelle 6 und Figur 5 beschreiben den Hydrierverlauf. Es gelang zu keinem Zeitpunkt des Versuchs die Jodzahl nach Härtung auf einen Wert unter 10 zu erniedrigen.

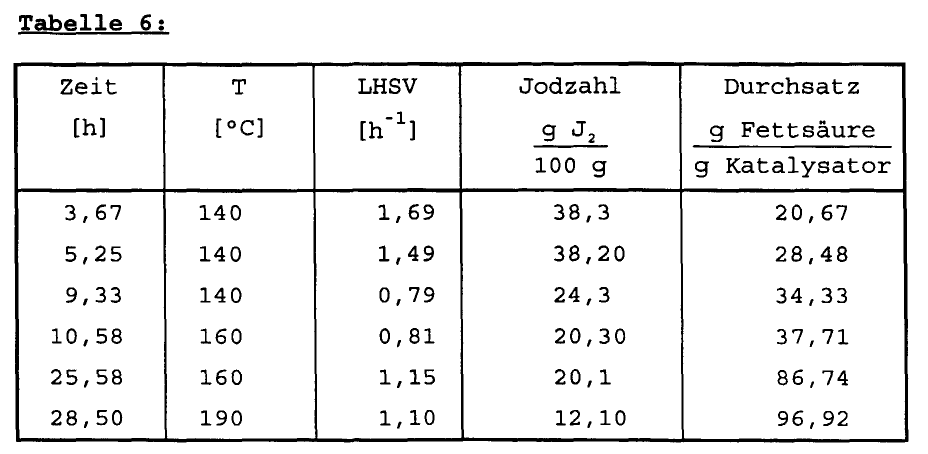

### Beispiel 2:

Als Ausgangsstoff für eine weitere Hydrierung diente reiner Linolsäureethylester. Auf eine der Hydrierung vorgeschaltete Extraktion konnte daher verzichtet werden. Die Hydrierung wurde in verdichtetem, unterkritischen Propan bei einem Gesamtdruck von 35 bar, einer Raumgeschwindigkeit von 60 h⁻¹ und einer Reaktionstemperatur von 60°C durchgeführt. Zum Einsatz kamen 2 ml des 1%-Pd/OFP Katalysators.

### Vergleichsbeispiel 4:

Beispiel 2 wurde mit einem auf 100 bar erhöhten Gesamtdruck wiederholt. Unter diesen Bedingungen befindet sich das als Fluid verwendete Propan in einem nahekritischen Zustand.

### Vergleichsbeispiel 5:

Vergleichsbeispiel 4 wurde mit Kohlendioxid anstelle von Propan wiederholt. Kohlendioxid befindet sich bei einem Gesamtdruck von 100 bar und einer Temperatur von 60°C in einem überkritischen Zustand.

### Vergleichsbeispiel 6:

Der Linolsäureethylester wurde in einer konventionellen Rieselbetthärtung, das heißt ohne Zugabe eines Fluids in reinem Wasserstoff bei einem Druck von 100 bar hydriert.

Die Ergebnisse von Beispiel 2 und der Vergleichsbeispiele 4 bis 6 sind in Tabelle 7 aufgelistet. Die gemessene Hydrieraktivität wurde wie in DE 44 05 029 beschrieben ermittelt.

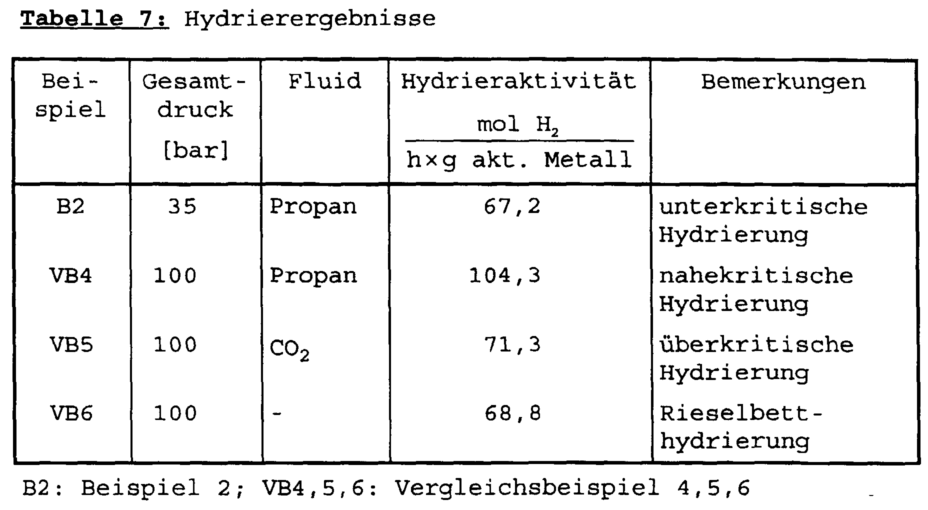

Beispiel 2 und die Vergleichsbeispiele 4 bis 6 zeigen deutlich, daß Fettrohstoffe wie Fette, Öle, Fettsäureester und freien Fettsäuren mit verdichteten, das heißt verflüssigten, Fluiden im unterkritischen Zustand hervorragend hydriert werden können. Die verdichteten Fluide werden hierbei als Lösungsmittel für die Fettrohstoffe eingesetzt. Hierfür eignen sich zum Beispiel die Fluide Kohlendioxid, Propan, Ethan, Butan, Distickstoffmonoxid oder Mischungen davon.

Wie Tabelle 7 zeigt, werden bei Lösung des Linolsäureethylesters in verdichtetem, unterkritischem Propan vergleichbare Hydrieraktivitäten wie in der konventionellen Rieselbetthärtung oder bei der überkritischen Hydrierung gemäß WO 95/22591, jedoch bei einem wesentlich verringerten Druck, erzielt.

Die Löslichkeit der eingesetzten Rohstoffe im Lösungsmittel kann auch hier durch den Einsatz von Modifikatoren erhöht werden. Das Reaktionsprodukt wird nach der Hydrierung durch Druckentspannung bzw. Temperaturerhöhung vom verdichteten, unterkritischen Fluid abgetrennt. Das Fluid wird gegebenenfalls nach Verdichtung beziehungsweise Temperaturabsenkung wieder in den Prozeß zurückgeführt. Zum Einsatz kommen die im Stand der Technik bekannten Hydrierkatalysatoren.

## Patentansprüche

1. Verfahren zur kontinuierlichen katalytischen Umsetzung einer oder mehrerer organischer Verbindungen, die zusammen mit unerwünschten Begleitstoffen einen Ausgangsstoff bilden, durch Extraktion der gewünschten organischen Verbindungen mittels verdichteter Fluide, gegebenenfalls Zugabe weiterer Reaktanden und weiterer Fluide zum Extrakt, welcher aus den verdichteten Fluiden und organischen Verbindungen besteht, Leiten des so erhaltenen Reaktionsgemisches über einen Katalysator zur katalytischen Umsetzung der organischen Verbindungen zu einem Produktgemisch aus den Einzelprodukten der katalytischen Umsetzung, Abtrennen des Produktgemisches vom Reaktionsgemisch und gegebenenfalls Rückführen der Fluide zur Extraktion.

2. Verfahren nach Anspruch 1,
**dadurch gekennzeichnet,**
daß Extraktion und katalytische Umsetzung bei Temperaturen zwischen 0 und 300°C und Drücken zwischen 10 und 800 bar vorgenommen werden.

3. Verfahren nach Anspruch 2,
**dadurch gekennzeichnet,**
daß die Extraktion im Gegenstrom mit verdichteten Fluiden aus der Gruppe Butan, Ethan, Kohlendioxid, Propan, Distickstoffmonoxid oder Mischungen davon vorgenommen wird, wobei die als Katalysatorgifte wirkenden unerwünschten Begleitstoffe zum überwiegenden Teil im Extraktionssumpf abgeschieden werden.

4. Verfahren nach Anspruch 3,
**dadurch gekennzeichnet,**
daß die Löslichkeit der organischen Verbindungen in den verdichteten Fluiden durch den Einsatz von Modifikatoren erhöht wird.

5. Verfahren nach Anspruch 4,
**dadurch gekennzeichnet,**
daß als Modifikatoren Aceton und/oder C₁ - C₆ Alkohole eingesetzt werden.

6. Verfahren nach einem der vorstehenden Ansprüche,
dadurch gekennzeichnet,
daß das Produktgemisch durch Druckverminderung beziehungsweise Temperaturerhöhung von den Fluiden abgetrennt wird.

7. Verfahren nach Anspruch 6,
**dadurch gekennzeichnet,**
daß das Produktgemisch bei der Abtrennung von den Fluiden durch fraktionierte Druckverminderung beziehungsweise Temperaturerhöhung in die Einzelprodukte aufgetrennt wird.

8. Verfahren nach einem der vorstehenden Ansprüche,
**dadurch gekennzeichnet,**
daß es sich bei den organischen Verbindungen um Fette, Öle, Fettsäureester und freie Fettsäuren handelt und die katalytische Umsetzung in einer Hydrierung besteht, wobei als weiterer Reaktand Wasserstoff zugeführt wird.

9. Verfahren nach Anspruch 8,
**dadurch gekennzeichnet,**
daß als Festbett-Hydrierkatalysatoren Platingruppenmetalle, Nickel oder Kupfer, gegebenenfalls zusammen mit Promotoren, auf geformten Trägern eingesetzt werden.

10. Verfahren zur selektiven oder vollständigen Hydrierung von Fetten, Ölen, Fettsäureestern und freien Fettsäuren durch Lösen dieser Stoffe in verflüssigten Gasen oder verflüssigten Gasgemischen und Hydrieren an einem Hydrierkatalysator in Gegenwart von Wasserstoff bei Temperaturen unterhalb der kritischen Temperatur für das verflüssigte Gas oder des verflüssigten Gasgemisches und Drücken zwischen 10 und 800 bar.
